(19) Europäisches Patentamt
European Patent Office
Office européen des brevets

(11) Numéro de publication: **0 040 153**
**B2**

(12) # NOUVEAU FASCICULE DE BREVET EUROPÉEN

(45) Date de publication du nouveau fascicule du brevet: **18.05.88**

(51) Int. Cl.⁴: **C 07 C 69/96**, C 07 C 68/02

(21) Numéro de dépôt: **81400743.1**

(22) Date de dépôt: **11.05.81**

(54) **Procédé de synthèse de chloroformiates alpha-chlorés et nouveaux chloroformiates alpha-chlorés.**

(30) Priorité: **14.05.80 FR 8010806**

(43) Date de publication de la demande: **18.11.81 Bulletin 81/46**

(45) Mention de la délivrance du brevet: **31.07.85 Bulletin 85/31**

(45) Mention de la decision concernant l'opposition: **18.05.88 Bulletin 88/20**

(84) Etats contractants désignés: **AT BE CH DE FR GB IT LI LU NL SE**

(56) Documents cité: **DE-C-121 223**

(73) Titulaire: **SOCIETE NATIONALE DES POUDRES ET EXPLOSIFS, 12, quai Henri IV, F-75181 Paris Cédex 04 (FR)**

(72) Inventeur: **Cagnon, Guy, 1, rue Gay- Lussac, F-91610 Ballancourt (FR)**
Inventeur: **Piteau, Marc, 38, avenue de Ballancourt, F-91710 Itteville (FR)**
Inventeur: **Senet, Jean- Pierre, 79, rue de la Gare Herbeauvilliers- Buthiers, F-77760 La Chapelle- la-Reine (FR)**
Inventeur: **Olofson, Roy A., 529 Cricklewood Drive, State College Pennsylvania 16801 (US)**
Inventeur: **Martz, Jonathan T., 1122 Center Lane, State College Pennsylvania 16801 (US)**

EP 0 040 153 B2

LIBER, STOCKHOLM 1988

**Description**

L'invention concerne la fabrication des chloroformiates α-chlorés et, à tire de produits industriels, de nouveaux chloroformiates α-chlorés.

La synthèse de chloroformiates α-chlorés de formule générale:

$$R-CHCl-O-\underset{\underset{O}{\|}}{C}-Cl$$

où R est un substituant aliphatique ou aromatique est une entreprise très difficile si l'on s'impose de ne pas additionner un atome de chlore supplémentaire sur le radical R, au cours de ladite synthèse.

Müller dans les Liebig's Annalen der Chemie de 1890, volume 257, page 50 et suivantes, a proposé un procédé qui est encore le seul connu et utilisé de nos jours. Ce procédé consiste à chlorer photolytiquement le chloroformiate correspondant, non substitué en α. On obtient malheureusement, à côté du produit recherché, de nombreux sous-produits plus chlorés que nécessaire. Müller en a ainsi dénombré pas moins de cinq dans le cas du chloroformiate d'éthyle qu'il a étudié.

Or, la présence de ces sous-produits est extrêmement gênante en raison de l'application principale qui est faite desdits chloroformiates à savoir leur transformation en carbonates notamment utiles en synthèse pharmaceutique fine.

Une distillation du produit de la réaction est donc indispensable bien que délicate en raison de la présence de nombreux sous-produits.

Il existe une autre publication ancienne, le brevet allemand 121 223 de 1901, décrivant la synthèse de chloroformiate de tétrachloro-1,2,2,2-éthyle et du chloroformiate d'α-chloro benzyle, par phosgénation respectivement du chloral et du benzaldéhyde, en présence d'une quantité stoechiométrique d'une amine tertiaire n'appartenant pas à la série pyridinique.

S'il vient à l'idée de tenter dans les mêmes conditions la phosgénation d'autres aldéhydes moins particuliers que les précédents, par exemple l'acétaldéhyde, on observe la formation de nombreux complexes et sous-produits à côté du chloroformiate d'α-chloréthyle qui n'est obtenu qu'avec un rendement médiocre, ce qui rend le procédé inintéressant à l'échelle industrielle.

Par ailleurs, s'il vient encore à l'idée de tenter la phosgénation avec une amine tertiaire aliphatique, par exemple la triéthylamine, on constate essentiellement la destruction de cette amine avec seulement formation d'une très faible quantité du chloroformiate dérivé.

Il existe donc un besoin non satisfait en un procédé de fabrication de chloroformiates α-chlorés purs, si possible avec un bon rendement, qui permette enfin d'assurer à ces produits de structure très simple le développement qu'ils méritent.

La demanderesse a maintenant trouvé un tel procédé de fabrication de chloroformiates α-chlorés exempts de sous-produits de substitution ultérieure à partir de matières premières peu onéreuses et conduisant à des rendements excellents. Ce procédé est le premier d'application aussi générale qui soit proposé depuis 90 ans.

L'invention consiste en un procédé de synthèse de chloroformiates alpha-chlorés de formule:

$$R \left( \underset{\underset{Cl}{|}}{CH} - O - \underset{\underset{O}{\|}}{C} - Cl \right)_m$$

dans laquelle R représente un reste hydrocarboné aliphatique, cycloaliphatique ou aromatique, substitué ou non substitué et m un nombre entier supérieur ou égale à un, caractérisé en ce que l'on fait réagir le phosgène sur l'aldéhyde correspondant $R\text{-(- }CHO)_m$ en présence d'un catalyseur choisi dans le groupe constitué par les amines tertiaires, les amides substituées, les urées substituées, les thiourées substituées, les phosphines tertiaires, les phosphoramides substituées, les produits de réaction de ces catalyseurs avec le phosgène, les halogénures d'oniums et les halogénures métalliques associés à un complexant de leur cation et en ce que la quantité de catalyseur utilisé est comprise entre 1 et 50 % en moles de catalyseur par rapport à la quantité molaire de fonction aldéhyde à transformer. C'est bien en cela que réside l'invention car, jusqu'à présent, depuis le début du siècle, on croyait que les aldéhydes n'étaient pas capables de réagir sur le phosgène si ce n'est que dans quelques cas très particuliers où on opérait en présence de quantités stoechiométriques de substances organiques basiques auxquelles on assignait en fait un rôle de complexant du phosgène.

On a pu trouver une définition commune à un certain nombre de catalyseurs convenant bien dans le cadre de l'invention. Ces catalyseurs sont des substances organiques ou minérales capables, dans un milieu contenant un aldéhyde de formule $R\text{-(-}CHO)_m$, du phosgène et, éventuellement, un solvant, de générer une paire d'ions dont l'un est un anion halogénure et l'autre un cation suffisamment séparé dudit anion halogénure pour impartir à ce dernier un pouvoir nucléophile lui permettant d'attaquer la ou les fonctions aldéhydes de la molécule $R\text{-(-}CHO)_m$. Comme catalyseurs selon l'invention entrant dans cette definition on peut notamment citer les substances suivantes en tant que telles ou sous forme de leur produit de réaction avec le phosgène:

les amines tertiaires, les amides substituées, les urées et thiourées substituées, les phosphines tertiaires, les phosphoramides substituées, les halogénures d'oniums tels que les halogénures d'ammoniums, de phosphoniums et d'arsoniums quaternaires, les halogénures de sulfoniums tertiaires et les halogénures métalliques associés à un complexant de leur cation. De préférence l'halogénure est le chlorure.

L'invention concerne également, à titre de produits industriels nouveaux, les nouveaux chloroformiates α-chlorés que permet d'obtenir le procédé selon l'invention, et qui sont particulièrement utiles comme agents de synthèse.

L'invention est donc remarquable à plusieurs égards: elle permet la phosgénation d'un grand nombre d'aldéhydes, elle démontre la possibilité d'effectuer cette phosgénation en présence de quantités catalytiques d'une très grande variété de substances.

Le procédé selon l'invention permet la phosgénation d'un grand nombre d'aldéhydes. Le procédé selon l'invention, lui, n'est pas largement influencé par la nature du radical R. Ceci ressort à la fois de constatations expérimentales et de considérations des mécanismes puisque la fonction aldéhyde est la principale concernée par la réaction et puisque R n'est ni intermédiairement ni finalement modifié. En revanche des facteurs tels que la taille de R peuvent avoir une influence sur certaines conditions opératoires sans que cela soit surprenant: par exemple la présence d'un R lourd amène à préférer une température de réaction supérieure au point de fusion de l'aldéhyde ou encore l'emploi d'un solvant pour l'aldéhyde.

Le radical R peut ainsi être un reste aliphatique ou cycloaliphatique, saturé ou non saturé, substitué ou non substitué. On peut ainsi phosgéner selon l'invention des aldéhydes aussi différents que l'aldéhyde acétique, le valéraldéhyde, le chloral, l'acroléïne, le cyclohexane carboxaldéhyde.

Le radical R peut également être un reste aromatique substitué ou non substitué.

De préférence quand R est un reste aliphatique, il comporte de 1 à 24 atomes de carbone. Quand R est un reste aliphatique insaturé, il comporte de préférence de 2 à 24 atomes de carbones. Quand R est un reste cycloaliphatique, il comporte de préférence de 3 à 24 atomes de carbone. Quand R est un reste aromatique, il comporte de préférence de 6 à 18 atomes de carbone dans les cycles aromatiques. Enfin, par reste substitué on entend de préférence un reste portant notamment un ou plusieurs atomes d'halogène ou un ou plusieurs groupes inertes vis à vis du phosgène ou donnant naissance à des groupes inertes par réaction avec le phosgène, tels que des groupes alkyle en $C_1$ - $C_{12}$, des groupes aryle, alkényle, alkynyle, aralkyle, des groupes $NO_2$, NRR', CN, OR, OH, COOR, COR, OCOOR, où R et R' sont un groupe hydrocarboné comportant de préférence de 1 à 12 atomes de carbone.

On peut ainsi phosgéner selon l'invention le benzaldéhyde, le chloro-2 benzaldéhyde, et l'aldéhyde téréphtalique. Le procédé selon l'invention s'applique aussi bien à des monoaldéhydes qu'à des polyaldéhydes.

Comme il a déjà été dit plus haut le procédé selon l'invention consiste à phosgéner, en présence ou non d'un tiers solvant, l'aldéhyde correspondant au chloroformiate α-chloré désiré en présence d'un catalyseur. Le terme "catalyseur" doit, dans la présente description, être pris dans une acceptation restrictive. Le composé ajouté en tant que catalyseur est indispensable au déroulement correct de la réaction, ne pas directement à la réaction et est utilisé en quantités relativement faibles par rapport à l'aldéhyde: en ce sens, il est bien un catalyseur; mais, contrairement à ce qui est communément admis pours les catalyseurs, il n'est pas toujours réutilisable pour une autre réaction une fois que l'on a arrêté l'arrivée de phosgène, et la demanderesse n'a pas d'explications théoriques à proposer pour ce phénomène.

Le taux de catalyseur employé est une caractéristique importante du procédé selon l'invention. En effet, s'agissant d'un catalyseur particulièrement efficace et d'un aldéhyde particulièrement réactif, un taux de 1 à 10 % en moles, de préférence de 3 à 7 %, de catalyseur par rapport à la quantité molaire de fonctions aldéhydes à transformer, doit être adopté. Par contre certains catalyseurs selon l'invention sont moins efficaces et un taux de 1 à 50 % environ, de préférence de 5 à 40 %, soit un taux en moyenne plus élevé, doit être utilisé. On peut dire que selon l'invention, il existe pour chaque catalyseur un taux maximum au delà duquel on constate que la réaction principale ne se fait plus ou s'accompagne d'importantes réactions secondaires. Ce taux maximum est d'autant plus faible que le catalyseur est efficace et d'autant plus élevé que le catalyseur est peu actif. Il est apparu que ce taux maximum se situe pour la plupart des catalyseurs entre 10 et 50 % environ en moles, par rapport à la quantité molaire de fonctions aldéhydes à transformer.

Un des aspects les plus frappants de la présente invention est l'extrême variété des substances susceptibles d'être employées comme catalyseurs de la réaction. La Demanderesse a essayé un très grand nombre de composés et a pu établir, d'une part, une liste de composés donnant de bons résultats et, d'autre part, un test permettant à l'homme de l'art de déterminer si un composé est un bon catalyseur au sens de la présente invention.

Comme il a été exposé plus haut un certain nombre de catalyseurs sont du type générateur d'anion halogénure soit directement, soit après réaction sur le phosgène.

Dans ce cas, le mécanisme général d'action du catalyseur est selon toute vraisemblance le suivant:

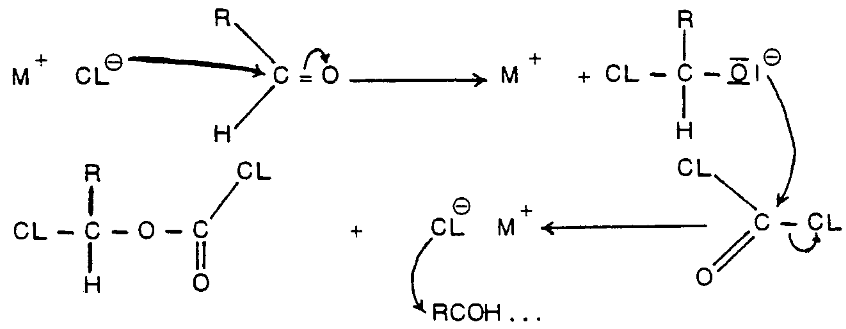

où $M^+$ représente un cation organique ou minéral, complexé ou non, présent en la forme dans le catalyseur dés le départ ou bien formé dès les premiers instants de la réaction par action du phosgène sur le catalyseur.

Ainsi $M^+$ peut être un cation métallique complexé ou un cation entièrement organique du type onium et on a par exemple:

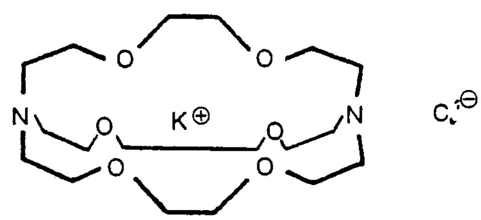

ou bien $M^+$ provient de la réaction plus ou moins avancée du phosgène sur la substance responsable de l'activité catalytique, comme par exemple dans la séquence:

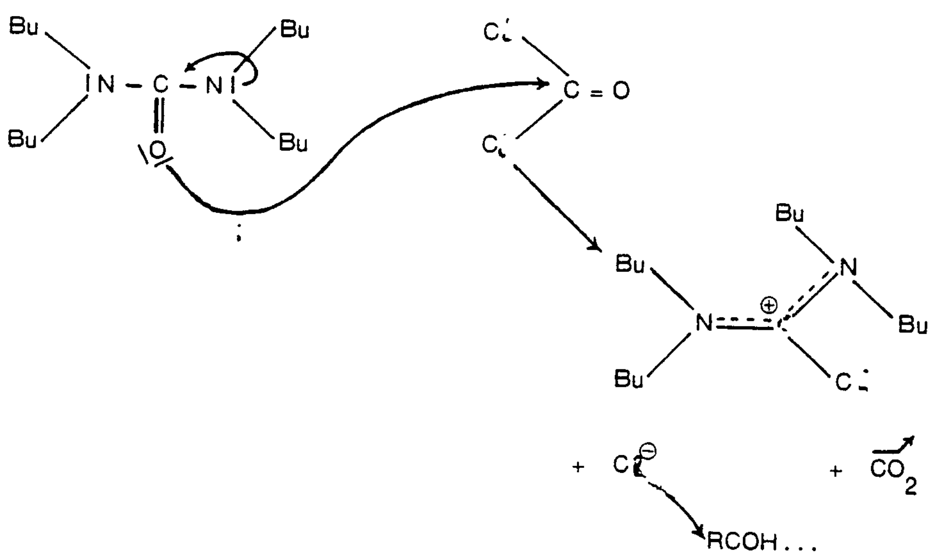

où donc $M^+$ est un volumineux cation chlorimonium.

On a observé que les résultats les plus intéressants sont obtenus avec les catalyseurs suivants: amines tertiaires aromatiques comportant un seul noyau aromatique, c'est-à-dire les monoarylamines tertiaires telles que la N,N-diméthylaniline, la N,N-diméthylaminopyridine et la cétone de Mischler ou di(paradiméthylaminophényl) cétone, les monoazines aromatiques y compris celles expréssément exclues par le brevet allemand 121 223 telles que la pyridine, les amines cycliques non aromatiques employées dans le brevet français 2 011 179 et notamment l'imidazole, les amides substituées et plus particulièrement la diméthylformamide, les urées et thiourées substituées et plus particulièrement les tétraalkyl (thio)urées telles que la tétrabutylurée et la tétraméthylurée, les phosphines tertiaires et notamment les phosphines tertiaires aliphatiques telles que la trioctyl phosphine, les phosphoramides substituées et plus particulièrement l'hexaméthylphosphotriamide, les halogénures d'ammonium, de phosphonium ou d'artsonium quaternaire et les halogènures de sulfonium tertiaire et notamment ceux où tous les radicaux hydrocarbyl substituants comportent tous réunis au moins 16 atomes de carbone et de préférence au moins 4 atomes de carbone

chacun, tels que le chlorure de tributyl benzyl ammonium, les halogénures métalliques associés à un complexant de leur cation, tels que les chlorures alcalins ou alcalins-terreux et notamment le chlorure de potassium associés à un éther couronne tel que le 18- couronne - 6 ou un cryptant tel que le (222) ou diaza-1, 10 hexaoxa-4, 7, 13, 16, 21, 24-bicyclo (8, 8, 8) hexacosane. Naturellement dans ce dernier cas on associe de préférence un complexant formant avec le cation du chlorure métallique un complexe ayant une constante de stabilité élevée ce ce qu'il est très facile de faire compte tenu des nombreuses études effectuées sur le sujet telles que celle de Kappenstein parue dans le Bulletin de la Société Chimique de France, 1974, N° 1-2, pages 89-109 et celle de J. M. LEHN parue dans Structure and Bonding, volume 16, pages 2-64, Springer Verlag, (1974). Les amines tertiaires aliphatiques sont, pour leur part, des catalyseurs moins actifs que les composés précédents, dans le cadre de la présente invention: à ce titre, elles n'apparaissent pas comme des catalyseurs préférés. Dans ce qui précède par halogénure on entend essentiellement un chlorure, un bromure ou un iodure, étant bien entendu qu'on préfère un chlorure, de manière à ce que même la première molécule d'aldéhyde transformée grâce à l'action de l'halogénure provenant du catalyseur, soit transformé en chloroformiate α-chloré.

Avec les catalyseurs du groupe des amides, des urées, des phosphines tertiaires ou avec la pyridine une phosgénation conduite entre 0 et 70°C donne déjà de bons résultats: c'est notamment le cas avec des catalyseurs comme les carboxamides tels que le diméthylformamide, les phosphoramides tels que l'hexaméthylphosphotriamide, les tétraalkylurées ou-thiourées telles que la tétrabutylurée, les phosphines tertiaires telles que la trioctylphosphine et bien sûr la pyridine. On choisira de préférence ces catalyseurs lorsque l'on souhaite pour certaines raisons, notamment la fragilité du chloroformiate α-chloré conduire la phosgénation à température modérée. Toutefois, si l'on opère à température plus élevée que 70°C, par exemple aux environs de 100°C, on observe généralement, avec ces mêmes catalyseurs, une élévation du rendement. Au-delà de 110°C, on encourt le risque de pyrolyse du chloroformiate α-chloré formé. En revanche, la réaction s'effectue en général bien avec les catalyseurs les plus efficaces, jusqu'à -10°C, température en dessous de laquelle la cinétique se ralentit assez rapidement.

Avec les amines tertiaires comportant un seul noyau aromatique telles que la N,N diméthylaminopyridine, la N,N diméthylaniline ou encore avec l'imidazole la phosgénation est préférentiellement conduite au delà de 70°C.

Un test pratique permettant à l'homme de l'art de dire qu'un composé est un bon catalyseur au sens de la présente invention consiste à regarder si on obtient un rendement supérieur à n % en chloroformiate d'α-chloroéthyle par réaction de quantités équimoléculaires d'acétaldéhyde et de phosgène, la réaction étant effectuée dans un tube fermé, dans le chlorobenzène, à 100°C ou dans le tétrachlorure de carbone à 40°C, sous agitation, en présence dudit composé à raison de n % en moles par rapport à l'acétaldéhyde, le rendement étant mesuré par rapport à l'acétaldéhyde de départ, au bout de 3 à 6 heures et n étant compris entre 5 et 50 et de préférence compris entre 5 et 15.

Il n'est pas nécessaire de pratiquer ce test sur une très grande échelle. Ainsi, on peut se contenter d'introduire, à 0°C, successivement 0,001 mole du composé pressenti, un barreau agitateur aimanté et 5 ml d'une solution à 2 moles/litre d'acétaldéhyde et à 2 mole/litre de phosgène dans le chlorobenzène dans un tube en verre de 20 ml, résistant à la pression, puis de sceller ledit tube et de le placer dans un bain thermostaté à 100°C en utilisant le barreau magnétique comme agitation constante et, enfin, après refroidissement à 0°C, de mesurer le rendement en chloroformiate d'α-chloroéthyle obtenu au bout de 3 heures, par Résonance Magnétique Nucléaire. Dans ce cas, on relève les valeurs d'intégration a, b, c et d du proton fixé au carbone en α des groupes méthyle des composés respectifs suivants: $CH_3CHO$, $CH_3CHClOCOCl$, $(CH_3CHO)_3$ (paraldéhyde) et $CH_3CHCl_2$, après quoi on regarde si le rapport 100 b/ a + b + c + d est supérieur ou égale à 15. Dans l'affirmative le composé testé est un bon catalyseur selon l'invention. A titre indicatif, on rappelle qu'aux protons susmentionnés correspondent respectivement des quadruplets à 9,7, 6,45, 4,9 et 5,85 ppm, la référence étant constituée par du TMS. Il est bien clair que le test précédent n'a qu'une valeur indicative et que certains catalyseurs que ne satisfont pas au test précédent sont néanmoins fort intéressants économiquement car, bien que relativement peu actifs, ils sont d'un prix modique. D'autres catalyseurs encore satisfont mal au test précédent car ils sont surtout actifs en l'absence de solvant: on peut citer par exemple KCl associé au (222).

Conformément à l'invention, la phosgénation est en générale conduite sous pression atmosphérique, mais dans certains cas il peut être intéressant d'opérer sous pression supérieure ou inférieure à la pression normale' par exemple dans le cas d'une phosgénation d'un aldéhyde volatile il pourra être utile d'opérer sous une pression légèrement supérieure à la normale.

La réaction peut être conduite dans un solvant inerte vis à vis du phosgène ou conduisant finalement, par réaction avec ce dernier, à un solvant inerte vis à vis du phosgène. Ce solvant est avantageusement choisi, notamment si le catalyseur n'est pas initialement du type ionique, parmi les solvants non polaires ou faiblement polaires et aprotiques tels que, par exemple, le tétrachlorure de carbone, le chloroforme, le dichlorométhane, le toluène, le chlorobenzène, l'hexane. Dans la mesure où l'on veut phosgéner un aldéhyde très réactif tel que par exemple l'acétaldéhyde, on choisit de préférence un solvant légèrement plus polaire que le tétrachlorure de carbone de manière à éviter le risque de formation de carbonate dichloré et on préfère ainsi par exemple le dichlorométhane; dans ce cas il peut également être intéressant de conduire la phosgénation à température relativement basse (35-40°C). Bien entendu, on peut également effectuer la réaction dans un milieu solvant constitué par le produit que l'on veut former lui-même. Ainsi on peut former un

pied de cuve à l'aide de chloroformiate α-chloré provenant d'une opération de fabrication antérieure et introduire ensuite réactifs et catalyseur. Une très bonne possibilité consiste à préparer un pied de cuve constitué du phosgène liquide et du catalyseur et, après réaction, à introduire ensuite l'aldéhyde et le reste du phosgène. Dans ce dernier cas la réaction débute en l'absence de solvant avant de se dérouler ensuite en solution dans le chloroformiate α-chloré progressivement formé.

Il a été observé que parfois la polarité du solvant a un effet d'autant plus positif sur le rendement qu'elle est plus élevée. Ceci est attribué au fait que dans un milieu polaire, l'anion halogénure et le cation du catalyseur sont mieux séparés que dans un milieu peu ou pas polaire, d'où une très grande réactivité dudit anion halogénure.

La réaction proprement dite de phosgénation se fait selon les techniques classiques connues de l'homme de métier. On peut ainsi soit mélanger le phosgène en solution dans une partie du solvant à une solution d'aldéhyde contenant le catalyseur ou encore faire barboter le phosgène gazeux dans une solution d'aldéhyde contenant le catalyseur. La phosgénation proprement dite dure plusieurs heures et est en générale conduite en milieu agité. Après phosgénation le chloroformiate a-chloré est en générale isolé du milieu réactionnel par distillation classique sous pression atmosphérique ou réduite. La demanderesse a observé à ce sujet que dans le cas de la synthèse de chloroformiates α-chlorés de la série benzylique on préfère utiliser comme catalyseur la pyridine avec le tétrachlorure de carbone comme solvant car, dans ces conditions, après phosgénation le catalyseur ou son produit de réaction avec le phosgène précipite ce qui permet d'isoler le chloroformiate α-chloré par simple filtration sans distillation.

Compte tenu de ce qui précède, il est clair que le procédé selon l'invention n'est pas seulement utilisable en discontinu. En effet, lorsque les meilleurs catalyseurs sont utilisés, les temps de réaction sont relativement courts et les quantités de chaleur dégagées par la réaction sont modestes: on peut donc opérer en continu et notamment utiliser des réacteurs en boucle, munis d'éjecteurs ou de convergents-divergents, le produit de la réaction étant, comme il a été dit, un bon milieu pour la réalisation de la réaction.

Les chloroformiates α-chlorés sont des agents de synthèse trés recherchés, notamment pour la synthèse pharmaceutique fine. L'invention concerne également, à titre de produits industriels, les nouveaux chloroformiates α-chlorés de formule

$$R_1 \!\leftarrow\! (\underset{\displaystyle Cl}{\underset{|}{CH}}-O-\underset{\displaystyle O}{\underset{\|}{C}}-Cl)_m$$

dans laquelle m représente un nombre entier pouvant prendre une valeur supérieure ou égale à un et $R_1$ représente:

- un reste aliphatique saturé, substitué ou non substitué, comportant au moins deux atomes de carbone, et au plus 24 atomes de carbone,
- un reste aliphatique mono ou poly insaturé substitué ou non substitué, en $C_2$-$C_{24}$,
- un reste cycloaliphatique substitué ou non substitué, en $C_3$-$C_{24}$
- un reste aromatique substitué.

Par reste substitué on entend un reste portant notamment un ou plusieurs atomes d'halogène ou un ou plusieurs groupes inertes vis à vis du phosgène ou donnant naissance à des groupes inertes par réaction avec le phosgène, tels que des groupes alkyle en $C_1$-$C_{12}$, des groupes aryle, alkényle, alkynyle, aralkyle, des groupes $NO_2$, NRR', CN, OR, OH, COOR, COR, OCOOR, où R et R' sont un groupe hydrocarboné.

L'invention concerne plus particulièrement les chloroformiates α-chlorés obtenus à partir des aldéhydes suivants:

- valéraldéhyde,
- acroléïne,
- chloro-2 benzaldéhyde,
- aldéhyde téréphtalique,
- cyclohexanecarboxaldéhyde.

C'est en effet un des avantages de la présente invention que de permettre l'obtention de chloroformiates α-chlorés nouveaux, non décrits jusqu'à présent dans la littérature, et que, dans certains cas, il est impossible d'obtenir par les procédés connus jusqu'à présent, comme en particulier les chloroformiates α-chlorés obtenus à partir d'aldéhydes insaturés.

Les exemples donnés ci-dessous illustrent l'invention sans en limiter la portée.

**Exemple 1**

Dans un réacteur de 500 ml équipé d'un agitateur, d'un thermomètre, d'un réfrigérant à carboglace et d'une ampoule de coulée, on place 44 g (1 mole) d'acétaldéhyde fraichement distillé, 200 ml de tétrachlorure de carbone anhydre et 120 g (1,2 mole) de phosgène. Le mélange étant maintenu à 0°C, on additionne en 15 minutes 28,4 g (0,1 mole) de tétra n-butylurée. La température est amenée à 40°C et la réaction est poursuivie

durant 2h30. Après élimination du phosgène en excès par dégazage et du solvant par évaporation, on obtient 72 g de chloroformiate de chloro-1 éthyle distillant à 117° C (la littérature indique 115-116° C), ce qui correspond à un rendement de 50 % en masse. La formule de produit étant:

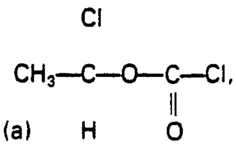

(a)

(b)

le spectre infra-rouge fait apparaître une bande à 1780 $cm^{-1}$ correspondant à la double liaison C=O tandis que le spectre RMN fait dans le chloroforme deutéré avec comme référence le tétraméthylsilane fait apparaître un doublet à 1,85 ppm correspondant aux protons (a) et un quadruplet à 6,44 ppm correspondant au proton (b)

**Exemple 2**

Cet exemple concerne la synthèse de chloroformiate d'α-chloréthyle à partir d'acétaldéhyde en présence d'hexaméthylphosphotriamide. Dans un réacteur en verre de 3 l muni d'un agitateur à ancre, d'un thermomètre, d'un réfrigérant à -35° C et d'un tube plongeant, on introduit:

- 1000 ml de dichlorométhane lavé à l'eau et séché sur sulfate de magnésium,
- 440 g (10 moles) d'acétaldéhyde brut anhydre,
- 179 g (1 mole) d'hexaméthylphosphorotriamide.

On refroidit le mélange à -5° C et introduit sous agitation, en 6h30 1107 g de phosgène gazeux.

On porte ensuite la température du milieu réactionnel à 35-40° C et maintient cette température pendant 3 heures.

On laisse reposer une nuit à température ambiante puis élimine le phosgène en excès par balayage à l'azote pendant 2h30.

On distille ensuite le mélange obtenu sous 150 mm de Hg dans une colonne en verre (hauteur 40 cm, diamètre 3 cm, garnissage hélices de Fenske de 0,5 cm) et recueille la fraction passant à 68° C.

On obtient ainsi 1020,4 g de chloroformiate d'α-chloroéthyle, ce qui correspond à un rendement de 71 % par rapport à l'acétaldéhyde engagé.

Analyse: - Spectre Infra-Rouge (C=O): 1780 $cm^{-1}$
- $n^{20}_D$ : 1,4220
- Densité $d^{15}_{15}$: 1,2946

**Exemple 3**

Cet exemple concerne la synthèse de chloroformiate d'α-chloréthyle à partir d'acétaldéhyde en présence de pyridine.

Dans un réacteur en verre de 500 ml équipé d'un agitateur à ancre, d'un thermomètre et d'un réfrigérant à reflux à acétone-carboglace, on introduit:

- 100 ml de dichlorométhane lavé à l'eau et séché sur sulfate de magnésium,
- 44 g (1 mole) d'acétaldéhyde brut anhydre,
- 79 g (0,1 mole) de pyridine fraîchement distillée.

On refroidit le mélange entre -5 et -10° C et ajoute en 1 h environ 120 g de phosgène.

On porte ensuite à léger reflux (température comprise entre 40 et 45° C) pendant 3h30.

On sépare les insolubles par filtration sous azote et distille le filtrat sous pression réduite. On obtient ainsi 90 g (rendement 63 %) de chloroformiate d'α-chloroéthyle (ébullition 68° C/150 mm Hg).

**Exemple 4**

Cet exemple concerne la synthèse du chloroformiate α-chloré obtenu à partir du valeraldéhyde.

Dans un réacteur de 100 ml équipé comme précédemment on introduit 21,5 g (0,25 mole) de n-pentanal, 50 ml de tétrachlorure de carbone et 1,9 g (0,025 mole) de pyridine. A ce mélange, refroidi à -5° C, on additionne 30 g (0,3 mole) de phosgène en 30 minutes. La température est amenée progressivement à 40° C. Après une heure à cette température le mélange réactionnel est dégazé à l'azote, filtré et distillé sous pression réduite. Le

chloroformiate d'α-chloro-n-pentyle distille à 73° C sous 15 mm de mercure.

Mass obtenue: 28 g ce qui correspond à un rendement de 60,5 %.

Spectre infra-rouge: C=O : 1790 $cm^{-1}$

$n^{20}_D$: 1,4377 densité (20° C): 1,1523

RMN ($CDCl_3$, TMS)

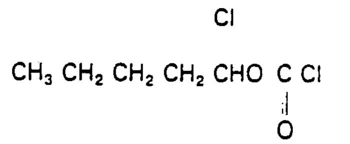

(a) (b) (c) (d)

(a) massif à 0,92 ppm (3H)

(b) massif à 1,40 ppm (4H)

(c) massif à 2,05 ppm (2H)

(d) triplet à 6,30 ppm (1H)

**Exemple 5**

Cet exemple concerne la synthèse du chloroformiate α-chloré obtenu à partir de l'acroléïne.

L'appareillage et le mode opératoire étant identique à ceux de l'exemple 4 on utilise les matières premières suivantes:

| | |
|---|---|
| - acroléïne (propenal) | : 28 g (0,5 mole) |
| - pyridine | : 3,95 g (0,05 mole) |
| -tétrachlorure de carbone | : 50 ml |
| - phosgène | : 60 g (0,6 mole) |

Le chloroformiate d'α-chloro allyle distille à 38° C sous 10 mm de mercure.

Mass obtenue: 42 g ce qui correspond à un rendement de 54 %

Spectre Infra-Rouge: C=O : 1780 $cm^{-1}$

$n^{20}_D$ : 1,4462 densité (20° C) : 1,2853

Spectre RMN:

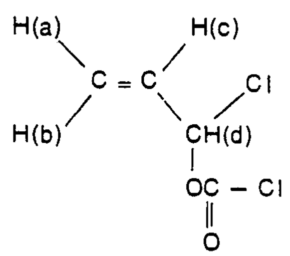

(a) (b) (c): massif complexe de 5,2 à 6,5 ppm (3H)

(d) : doublet à 6,71 ppm (1H)

**Exemple 6**

Cet exemple concerne la synthèse du chloroformiate α-chloré obtenu à partir du benzaldéhyde.

Les produits utilisés sont les suivants:

| | |
|---|---|
| - benzaldéhyde | : 26,5 g (0,25 mole) |
| - pyridine | : 1,95 g (0,025 mole) |
| - phosgène | : 35 g (0,35 mole) |
| - tétrachlorure de carbone | : 50 ml |

En utilisant un mode opératoire identique à celui de l'exemple 4 on obtient 34,8 g (68 %) de chloroformiate d'α-chlorobenzyle distillant à 70° C sous 0,4 mm de mercure.
spectre infra-rouge: C=O: 1770 $cm^{-1}$
$n^{20}_D$: 1,5367 densité (20°): 1,3016
spectre RMN:

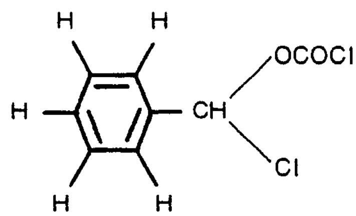

tous les protons ont un déplacement chimique compris entre 7 et 8 ppm.

**Exemple 7**

Cet exemple concerne la synthèse du chloroformiate α-chloré obtenu à partir de chloro-2 benzaldéhyde.
Par rapport à l'exemple 6 on remplace le benzaldéhyde par le chloro-2 benzaldéhyde.
On obtient 25,1 g (rendement: 42 %) de chloroformiate d'α-chloro (chloro-2) benzyle distillant à 85-88° C sous 0,2 mm de Hg.
spectre infra-rouge: C=O: 1780 $cm^{-1}$
$n^{20}_D$: 1,5420 densité (20° C) 1,4294
spectre RMN:

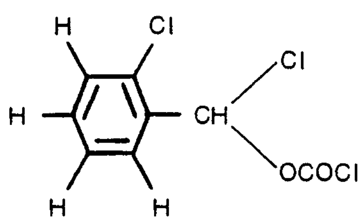

tous les protons ont un déplacement chimique compris entre 7 et 8 ppm.

**Exemple 8**

Cet exemple concerne la synthèse du chloroformiate α-chloré obtenu à partir de l'aldéhyde téréphtalique.
Dans un réacteur de 500 ml on place 67 g (0,5 mole) d'aldéhyde téréphtalique, 3,95 g (0,05 mole) de pyridine et 100 ml de tétrachlorure de carbone. On introduit ensuite à 0° C 120 g (1,2 mole) de phosgène.
Le mélange est alors porté progressivement à 40° C et maintenu à cette température durant 3 heures. Après dégazage, filtration, et élimination du solvant on obtient 133 g (rendement: 80 %) d'une huile incolore.
taux de chlore total (en %): calculé: 42,7 trouvé: 40,02
spectre IR: C=O 1780 $cm^{-1}$
spectre RMN
($CDCl_3$, TMS)

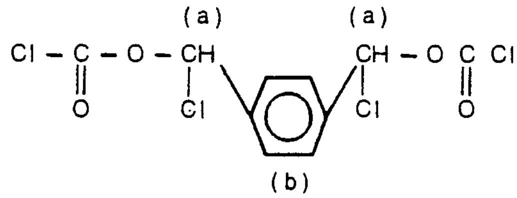

(a) singulet à 7,29 ppm (2H)
(b) singulet à 7,64 ppm (4H)

**Exemple 9**

Cet exemple concerne la synthèse de chloroformiate d'α-chloroéthyle à partir de l'acétaldéhyde en présence de trioctyl phosphine.

Dans un réacteur de 100 ml on place 11 g (0,25 mole) d'acétaldéhyde, 9,25 g (0,025 mole) de trioctylphosphine et 50 ml de tétrachlorure de carbone. A ce mélange refroidi à 0°C on additionne 30 g (0,3 mole) de phosgène. Aprés 1 heure de chauffage à 35-40°C le mélange réactionnel est dégazé et distillé sous pression réduite (150 mm de Hg). On obtient ainsi 9,1 g (rendement: 25 %) de chloroformiate d'α-chloroéthyle distillant à 67-68°C.

**Exemple 10**

Cet essai concerne la préparation du chloroformiate d'α-chloroéthyle par phosgénation de l'acétaldéhyde en présence de 5 % en mole de pyridine en milieu solvant.

Le mode opératoire et l'appareillage sont identiques à ceux de l'exemple 4.

Les quantités de produits utilisés sont les suivantes:

| | |
|---|---|
| - acétaldéhyde | : 11 g (0,25 mole) |
| - pyridine | : 0,99 g (0,0125 mole) |
| - phosgène | : 30 g (0,3 mole) |
| - chlorure de méthylène | : 50 ml |

On obtient ainsi 25,6 g (rendement 71,6 %) de chloroformiate d'a-chloroéthyle distillant à 68°C sous 150 mm de mercure.

**Exemple 11**

Cet essai concerne la préparation du chloroformiate d'α-chloréthyle par phosgénation de l'acétaldéhyde en présence de 5 % en mole de pyridine sans solvant.

Dans un réacteur de 100 ml équipé comme dans les exemples précédents, on place à 0°C, 22 g (0,5 mole) d'acétaldéhyde et 1,98 g (0,025 mole) de pyridine. On introduit à cette température 60 g (0,6 mole) de phosgène. On porte en quatre heures le mélange réactionnel à 30°C et maintient à cette température durant 1 heure. Après élimination du phosgène on obtient 42,1 g (rendement 59 %) de chloroformiate d'α-chloroéthyle distillant à 68°C sous 150 mm de mercure.

**Exemple 12**

Cet essai concerne la phosgénation du cyclohexyle carboxaldéhyde.

Dans un réacteur de 100 ml on place 28 g (0,25 mole) de cyclohexyle carboxaldéhyde, 1,98 g (0,025 mole) de pyridine et 50 ml de tétrachlorure de carbone. Ce mélange étant refroidi à 0°C on introduit 30 g (0,3 mole) de phosgène. Le milieu réactionnel est ensuite porté à 35-40°C et maintenu durant 1 heure à cette température.

Après dégazage, filtration et élimination sous pression réduite du solvant on obtient 46 g (rendement 87 %) de chloroformiate attendu distillant à 90-93° C sous 10 mm de mercure.
$n^{20}_D$. 1,4738 densité (20° C): 1,1934
Spectre RMN ($CDCl_3$, TMS)

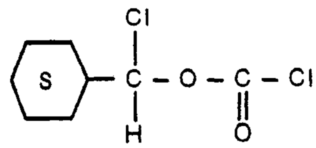

(a) (b) (a) massif de 1,15 à 2,2 ppm (11H)
(b) doublet a 6,1 ppm (1H)

**Exemples 13 à 17**

Ces essais, réalisés avec l'appareillage de l'exemple 4, ont pour but d'illustrer l'efficacité de divers catalyseurs en fonction de la température.
Chacun de ces essais a été effectué sur les quantités suivantes:

| | |
|---|---|
| - acétaldéhyde | : 4,4 g (0,1 mole) |
| -toluène | : 45 g |
| - catalyseur | : 0,01 mole |
| - phosgène | : 12 g (0,12 mole) |

Les résultats obtenus en fonction de la température après 3 heures de réaction sont portés dans le tableau ci-dessous, étant précisé que la notion de réaction ou d'absence de réaction est appréciée par spectrophotométrie infra-rouge. L'absence de réaction signifie que le rendement est inférieur à 5 %.

| EXEMPLE No | CATALYSEURS | 40° C | 70° C | 100° C |
|---|---|---|---|---|
| 13 | N,N diméthylaminopyridine | pas de réaction | pas de réaction | réaction |
| 14 | N,N dimethylaniline | pas de réaction | réaction | - |
| 15 | imidazole | pas de réaction | pas de réaction | réaction |
| 16 | produit de reaction de la tetra n-butylurée sur le: phosgene 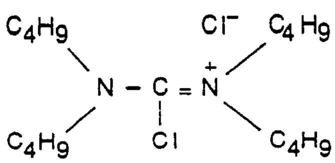 | réaction | - | - |
| 17 | dimethylformamide | réaction | | |

**Exemples 18 à 26**

On a réalisé deux séries d'essais de catalyseurs selon l'invention en vue de la synthèse de chloroformiate d'α-chloroéthyle.

Ces deux séries ne différent que par la température de réaction et le solvant utilisé.

A 0°C, on a placé successivement dans un tube scellable de 20 ml en verre résistant à la pression, 0,001 mole de catalyseur, un barreau agitateur aimanté, et 5 ml d'une solution à 2 moles/l d'acétaldéhyde et 5 ml d'une solution à 2 moles/l de phosgène dans le tétrachlorure de carbone (série 1) ou dans le chlorobenzène (série 2).

La quantité de catalyseur était ainsi de 10 % en mole par rapport à l'acétaldéhyde.

On a ensuite rapidement scellé le tube qu'on a placé dans un bain thermostaté à (40°C (série 1) ou à 100°C (série 2).

L'agitation transmise par le barreau aimanté étant mise en marche, on a laissé la réaction s'effectuer pendant 3 heures. Au bout de cette période on a refroidi le tube à 0°C environ, on a prélevé un échantillon du milieu réactionnel qu'on a aussitôt analysé par RMN. (référence TMS). La réaction étant effectuée en vase clos, il n'y a pas de perte d'acétaldéhyde qui se retrouve sous l'une des 4 formes suivantes:

$CH_3CH_aO$: à $H_a$ correspond un quadruplet à 9,7 ppm dont l'intégration a une valeur a.

$CH_3CH_bOCOCl$: à $H_b$ correspond un quadruplet à 6,45 ppm dont l'intégration a une valeur b.
Cl

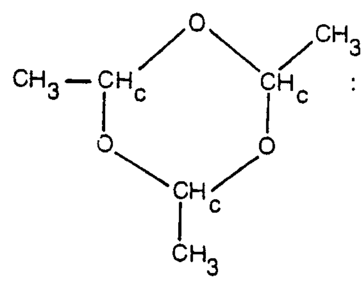

à $H_c$ correspond un quadruplet à 4,9 ppm dont l'intégration a une valeur c.

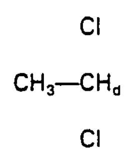

à $H_d$ correspond un quadruplet à 5,85 ppm dont l'intégration a une valeur d.

Les rendements en chloroformiate d'α-chloroéthyle obtenus avec chacun des catalyseurs ont été déterminés à l'aide de la formule:

$$\frac{100b}{a+b+c+d}$$

| Catalyseurs<br>Rendement Série | TBU (a) | Pyridine | TMU (b) | Quinoléine | CM (c) |
|---|---|---|---|---|---|
| Rendement en %<br>série 1 ($CCl_4$, 40°C) | 45 | 100 | 45 | 10 | 15 |
| Rendement en %<br>Série 2 (Chlorobenzène, 100°C) | 75 | - | 75 | 65 | 65 |

(a) Tétrabutylurée
(b) Tétraméthylurée
(c) Cétone de Mischler

**Exemple 27**

Dans un keller de 50 ml équipé d'une agitation magnétique et surmonté d'une condenseur à acétone carboglace maintenu à -50°C, on a introduit: 1,30 g (0,0175 mole) de KCl, 0,40 g (0,00106 mole) de kryptofix (222), commercialisé par Merck, 12,5 g (0,125 mole) de phosgène et finalement, 2,2 g (0,05 mole) d'acétaldéhyde.

On a agité le mélange réactionnel pendant 5 heures à température ambiante (18-22°C). A l'issue de cette période, l'analyse du milieu par RMN démontre que le rendement en chloroformiate d'α-chloréthyle est de 96 %, le résidue étant pratiquement uniquement constitué par de l'acétaldéhyde.

On trouvera une liste de complexant et notamment de cryptants utilisables comme le (222) précédent, quoiqu'éventuellement en association avec un sel plus adapté tel que NaCl, dans la revue Kontakte éditée par la Société Merck, 1/77, page 11-31. On appréciera particulièrement l'association de NaCl et du polymère kryptofix (222 B) constituant un catalyseur insoluble fonctionnant sans solvant.

**Exemple 28**

Dans un réacteur Claisen de 50 ml on a introduit 0,5 g de bromure de tétra-n-hexyl ammonium, 6,25g de phosgène et 1,1 g d'acétaldéhyde. On a laissé le mélange réactionnel sous agitation pendant 4 heures à température ambiante (18-20°C). Au bout de ce temps, on a fait un spectre RMN du mélange et on a observé qu'il ne contient que du chloroformiate d'α-chloro éthyle, soit un rendement remarquable de 100 %.

**Exemples 29 et 30**

On a repris le mode opératoire de l'exemple 28, en utilisant 5 % en mole, par rapport à l'acétaldéhyde, de chlorure de benzyl tri-n-butyl ammonium et de chlorure de méthyl tri-n-octyl ammonium. On a obtenu respectivement dans le premier cas 100 % de chloroformiate α-chloré, dans le second cas 12 % seulement, plus 33 % d'acétaldéhyde et 55 % de paraldéhyde.

La grosseur de l'anion et l'encombrement du cation apparaissent comme des facteurs favorables. On préfère largement les halogénures d'ammonium aux autres sels d'oniums.

On notera ainsi qu'il est extrêmement difficile d'obtenir un chloroformiate α-chloré avec l'aide du chlorure de tétraméthyl ammonium et qu'alors que le bromure de tétrahexylammonium donne d'excellents résultats (exemple 28), le chlorure correspondant est bien moins bon et à peu près comparable au bromure de tétrabutyl ammonium.

**Revendications**

1. Procédé de synthèse de chloroformiates alpha-chlorés de formule

$$R\left(\underset{\displaystyle Cl}{\underset{|}{CH}} - O - \underset{\displaystyle O}{\underset{\|}{C}} - Cl\right)_m$$

dans laquelle:

R représente un reste hydrocarboné, substitué ou non substitué,

m représente un nombre entier égal ou supérieur à un, par réaction du phosgène sur un aldéhyde de formule $R\text{-(-CHO)}_m$

dans laquelle R et mont la même signification,

caractérisé en ce que la réaction est effectuée en présence d'un catalyseur choisi dans le groupe constitué par les amines tertiaires, les amides substituées, les urées substituées, les thiourées suhstituées, les phosphines tertiaires, les phosphoramides substituées, les produits de réaction de ces catalyseurs avec le phosgène, les halogénures d'oniums et les halogénures métalliques associés à un complexant de leur cation et en ce que la quantité de catalyseur utilisé est comprise entre 1 et 50 % en moles de catalyseur par rapport à la quantité molaire de fonctions aldéhyde à transformer.

2. Procédé selon la revendication 1 caractérisé en ce que le dit halégonure d'onium est un halogénure d'ammonium quaternaire.

3. Procédé selon la revendication 1 caractérisé en ce que le dit catalyseur est un halogénure de métal alcalin ou alcalino-terreux associé à un éther couronne ou un cryptant.

4. Procédé selon l'une quelconque des revendications 2 ou 3 caractérisé en ce que le dit halogénure est un

chlorure.

5. Procédé selon l'une quelconque des revendications 1 à 4 caractérisé en ce que la quantité de catalyseur utilisé est comprise entre 3 et 7 % en moles de catalyseur par rapport à la quantité molaire de fonctions aldéhyde à transformer.

6. Procede selon l'une quelconque des revendications 1 à 5 caractérisé en ce que la réaction est conduite entre - 10° C et 110° C, de préférence entre 0 et 70° C.

7. Procédé selon l'une quelconque des revendications 1 à 6 caractérisé en ce que la réaction est conduite dans un solvant aprotique inerte vis à vis du phosgène, non polaire ou faiblement polaire.

8. Procédé selon la revendication 7 caractérisé en ce que le solvant utilisé est constitué par le produit de la réaction lui-mêne.

9. Procédé selon l'une quelconque des revendications 1 à 6 caractérisé en ce que la réaction est effectuée en l'absence de tiers solvant.

10. A titre de produits industriels nouveaux, les chloroformiates alpha-chlorés de formule

$$R_1 \leftarrow\!\!\!\!+ \underset{\underset{Cl}{|}}{CH} - O - \underset{\underset{O}{\|}}{C} - Cl)_m$$

dans laquelle:

m représente un nombre entier pouvant prendre une valeur supérieure ou égale à un et $R_1$ représente:
- un reste aliphatique saturé, substitué ou non substitué, comportant au moins deux atomes de carbone,
- un reste aliphatique insaturé substitué ou non substitué,
- un reste cycloaliphatique substitué ou non substitué,
- un reste aromatique substitué,.

11. Nouveau chloroformiates alpha-chlorés selon la revendication 10 caractérisé en ce qu'ils sont obtenus à partir des aldéhydes suivants:
- valéraldéhyde,
- acroléïne,
- chloro-2 benzaldéhyde,
- aldéhyde téraphtalique,
- cyclohexane carboxaldéhyde.

**Patentansprüche**

1. Verfahren zur Synthese von α-chlorierten Chlorameisensäureestern der Formel

$$R \leftarrow\!\!\!\!+ \underset{\underset{Cl}{|}}{CH} - O - \underset{\underset{O}{\|}}{C} - Cl)_m$$

in der R ein gegebenenfalls substituierter Kohlenwasserstoffrest und m eine ganze Zahl $\geqq$ 1 ist, durch Umsetzung von Phosgen mit einem Aldehyd der Formel R -(-CHO)$_m$,

in der R und m die gleiche Bedeutung haben, dadurch gekennzeichnet, daß die Reaktion in Gegenwart eines Katalysators, ausgewählt unter tertiären Aminen, substituierten Amiden, substituierten Harnstoffen, substituierten Thioharnstoffen, tertiären Phosphinen, substituierten Phosphoramiden, den Reaktionsprodukten dieser Katalysatoren mit Phosgen, Oniumhalogeniden und Metallhalogeniden, die an einen Komplexbildner für ihr Kation assoziiert sind, in einer Menge von 1 bis 50 Mol-%, bezogen auf die molare Menge an umzuwandelnder Aldehydfunktion, durchgeführt wird.

2. Verfahren nach Anspruch 1, dadurch gekennzeichnet, daß das Oniumhalogenid ein quartäres Ammoniumhalogenid ist.

3. Verfahren nach Anspruch 1, dadurch gekennzeichnet, daß der Katalysator ein an einen Kronenether oder Kryptanden assoziiertes Alkali- oder Erdalkalimetallhalogenid ist.

4. Verfahren nach Anspruch 2 oder 3, dadurch gekennzeichnet, daß das Halogenid ein Chlorid ist.

5. Verfahren nach einem der Ansprüche 1-4, dadurch gekennzeichnet, daß die verwendete Katalysatormenge von 3 bis 7 Mol-%, bezogen auf die molare Menge an umzuwandelnder Aldehydfunktion, beträgt.

6. Verfahren nach einem der Ansprüche 1-5, dadurch gekennzeichnet, daß die Reaktion bei -10 bis 110° C und vorzugsweise von 0 bis 70° C durchgeführt wird.

7. Verfahren nach einem der Ansprüche 1-6, dadurch gekennzeichnet, daß die Reaktion in einem gegenüber Phosgen inerten, aprotischen, nicht polaren oder schwach polaren Lösungsmittel durchgeführt wird.

8. Verfahren nach Anspruch 7, dadurch gekennzeichnet, daß das verwendete Lösungsmittel das Reaktionsprodukt selbst ist.

9. Verfahren nach einem der Ansprüche 1-6, dadurch gekennzeichnet, daß die Reaktion in Abwesenheit eines weiteren Lösungsmittels durchgeführt wird.

10. α-chlorierte Chlorameisensäureester der Formel

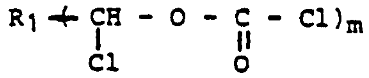

in der

m eine ganze Zahl ≥ 1 sein kann und $R_1$ bedeutet:

- einen gesättigten, gegebenenfalls substituierten aliphatischen Rest mit mindestens zwei Kohlenstoffatomen,
- einen ungesättigten, gegebenenfalls substituierten aliphatischen Rest,
- einen gegebenenfalls substituierten cycloaliphatischen Rest,
- einen substituierten aromatischen Rest.

11. α-chlorierte Chlorameisensäureester nach Anspruch 10, dadurch gekennzeichnet, daß sie von den folgenden Aldehyden ausgehend erhalten werden:

- Valeraldehyd
- Acrolein,
- 2-Chlorbenzaldehyd,
- Terephthalaldehyd,
- Cyclohexancarboxaldehyd.

## Claims

1. Pprocess for the synthesis of alpha-chlorinated chloroformates of the formula

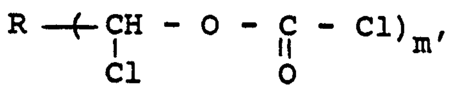

in which: R represents a substituted or unsubstituted hydrocarbon radical,

m represents an integer equal to or greater than one, by reaction of phosgene with an aldehyde of formula $R\text{-}(\text{-}CHO)_m$, in which R and m have the same meaning as above, characterised in that the reaction is carried out in the presence of a catalyst chosen from the group comprising tertiary amines, substituted amides, substituted ureas, substituted thioureas, tertiary phosphines, substituted phosphoramides, the reaction products of these catalysts with phosgene, onium halides and metal halides associated with a complexant of their cation and in that the quantity of catalyst used is between 1 and 50 moles %, relative to the molar quantity of aldehyde functions to be transformed.

2. Process according to Claim 1 characterised in that said onium halide is a quaternary ammonium halide.

3. Process according to Claim 1 characterised in that said catalyst is an alkali metal or alkaline earth metal halide associated with a crown ether or a cryptant.

4. Process according to any one of Claims 2 or 3 characterised in that said halide is a chloride.

5. Process according to any one of Claims 1 to 4 characterised in that the quantity of catalyst used is between 3 and 7 moles % of catalyst relative to the molar quantity of aldehyde functions to be transformed.

6. Process according to any one of Claims 1 to 5 characterised in that the reaction is carried out at between -10 and 110° C, preferably between 0 and 70° C.

7. Process according to any one of Claims 1 to 6 characterised in that the reaction is carried out in a nonpolar or weakly polar aprotic solvent which is inert towards phosgene.

8. Process according to Claim 7 characterised in that the solvent used consists of the reaction product itself.

9. Process according to any one of Claims 1 to 6 characterised in that the reaction is carried out in the absence of a third solvent.

10. By way of new industrial products, the alpha-chlorinated chloroformates of the formula

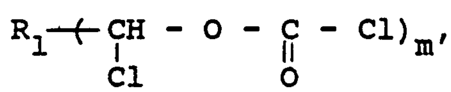

in which:

m represents an integer which can have a value greater than or equal to one and $R_1$ represents:

- a substituted or unsubstituted saturated aliphatic radical, containing at least two carbon atoms,
- a substituted or unsubstituted unsaturated aliphatic radical,
- a substituted or unsubstituted cycloaliphatic radical,
- a substituted aromatic radical.

11. New alpha-chlorinated chloroformates according to Claim 10, characterised in that they are obtained from the following aldehyde:

- valeraldehyde,
- acrolein,
- 2-chlorobenzaldehyde,
- terephthalaldehyde,
- cyclohexanecarboxaldehyde.